# EUROPEAN PATENT SPECIFICATION

(11) **EP 4 384 079 B1**
(45) Date of publication and mention of the grant of the patent: **30.10.2024**
(21) Application number: 22761246.2
(22) Date of filing: 10.08.2022
(51) Int. Cl.: A61B 8/08, A61B 8/00, A61B 5/107, A61B 6/00, A61B 5/055

(54) **APPARATUS AND METHOD FOR PROCESSING IMAGE DATA**
VORRICHTUNG UND VERFAHREN ZUR VERARBEITUNG VON BILDDATEN
APPAREIL ET PROCÉDÉ DE TRAITEMENT DE DONNÉES D'IMAGE

(30) Priority: 13.08.2021 WO PCT/CN2021/112504; 24.09.2021 EP 21198768
(43) Date of publication of application: 19.06.2024
(73) Proprietor: Koninklijke Philips N.V., 5656 AG Eindhoven (NL)
(72) Inventor: HONG, Xiaowei, 5656 AG Eindhoven (NL); MENG, Yishuang, 5656 AG Eindhoven (NL); XU, Jing Ping, 5656 AG Eindhoven (NL)
(74) Representative: Philips Intellectual Property & Standards
(86) International application number: PCT/EP2022/072472
(87) International publication number: WO 2023/017089

(56) References cited:
- WO-A1-2021/051855
- US-A1- 2016 004 330
- US-B2- 9 072 489

## Description

### FIELD OF THE INVENTION

The invention relates to an apparatus and method for use in processing image data, and more particularly, relates to processing image data relating to a subject.

### BACKGROUND OF THE INVENTION

Pelvic floor dysfunctions (PFDs) is a common medical condition that affects one third of women in China and one quarter of women in the United States. The disease is related to abnormal spasms or control issues in the muscle that support the pelvic organs. Common symptoms of pelvic floor dysfunctions include pelvic organ prolapse, urinary incontinence and faecal incontinence.

In spite of the prevalence of pelvic floor dysfunction, the condition is often not reported by patients until severe symptoms have shown. Recently, post-partum screening has become more common to diagnose the condition in the earlier stages. Physical examination is widely used as an initial approach for assessing PFDs, and can be used to diagnose approximately 50% of conditions. However, results from physical exams are highly subjective and descriptive, experience-dependent, and have limited use for determining the severity of the disease. In order to address this, imaging methods such as ultrasound are becoming more widely used to assess a subject.

Ultrasound and magnetic resonance (MR) are two commonly used imaging modalities for assessing pelvic floor structures and functions. Ultrasound typically serves as the first-line imaging modality. Compared to MR, ultrasound has the advantages of real-time imaging, and is widely available, low cost, and easy-to-use, which are beneficial in PFD diagnosis. Observations and measurements of the movement of pelvic organs during muscle contraction and Valsalva maneuver are important for assessing pelvic organ prolapse and have direct indication for urinary incontinence, fecal incontinence and other symptoms.

Pelvic floor ultrasound (PFUS) has proven useful for evaluating the pelvic floor including the urethra, bladder, vagina, cervix, anorectum, levator ani and sphincter. Both descriptive observations and quantitative measurements are taken during a PFUS examination for a comprehensive assessment of the pelvic floor of a subject.

WO2021/051855A1 disclosed a pelvic floor measurment method where pelvic floor key part points can be automatically recognized on the utrasound pelvic floor image. US9072489B2 disclosed an input unit that inputs feature points of a target part and a contour extraction unit that extracts a contour along the shape of the target part using the feature points. US2016/004330A1 disclosed a handheld ultrasound imaging apparatus, including a display and a pluarity of user inerface objects, wherein the pointer of the user interfance can be set as a caliper for distance measurement.

### SUMMARY OF THE INVENTION

The current methods available for assessing pelvic floor ultrasound images involve a clinician identifying relevant points corresponding to well-defined landmarks, or anatomical features or sub-features, in an ultrasound image which may then be used to perform a measurement. The clinician may click on the identified points and indicate the points that have been identified to an interface, along with a desired measurement, so that a computing device (or, for example, software) can then make the calculation or perform the measurement using the points. However, a user may be required to identify the same point corresponding to the same anatomical feature to make a new measurement, even if the point has been previously used to perform a different measurement, and the desired measurement must also be indicated along with the identified points. Furthermore, current methods may require a user to indicate points in a particular order.

In other existing systems, the clinician may be required to indicate points in a specific order shown on the user interface to take a measurement corresponding to a particular workflow. Such interfaces may not allow the clinician to skip or change the order of the inputs required for the measurements. However, in a routine workflow, the clinician may not always need to measure all the parameters set by an interface. Furthermore, these systems may only allow a limited number of measurements to be performed with a limited number of identified points.

It is therefore desirable to improve the efficiency of the processes by which images relating to a pelvic floor of a subject can be analysed, and to improve the flexibility of such processes.

According to an aspect, there is provided an apparatus for use in processing image data relating to a subject, the apparatus comprising a memory comprising instruction data representing a set of instructions; and a processor configured to communicate with the memory and to execute the set of instructions, wherein the set of instructions, when executed by the processor, cause the processor to, responsive to receiving input indicating a point in an image corresponding to an anatomical feature or sub-feature of the subject, determine if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and if sufficient points to perform a measurement have been received, perform the measurement.

Advantageously, this enables a more efficient process whereby measurements are performed automatically once all features and/or sub-features required to perform a measurement have been identified. The proposed solution may perform measurements automatically once all the required points have been collected, and may use the same point for different measurements where possible. This reduces the time taken for measurements to be obtained.

The pre-stored anatomical data may therefore be a data set which comprises a list of anatomical features or sub-feature, where groups of features from the list may be usable, or required, to perform particular measurements. It will be appreciated that the same anatomical feature or sub-feature may be used to perform different measurements, e.g. the same anatomical feature or sub-feature may be used to perform more than one measurement. The determining may involve comparing any received points (e.g. any points that have been indicated by the user for the same image data) to the pre-stored anatomical data to determine if the points which have been indicated can be used to perform a measurement, for example each time a new point is indicated.

The processor may be further caused to determine to which anatomical feature or sub feature the point corresponds based on received input from a user indicating the anatomical feature or sub feature to which the point corresponds, or by classifying the point into a class corresponding to the anatomical feature or sub feature.

The processor may be further caused to determine to which anatomical feature or sub feature the point corresponds by classifying the point into a class corresponding to an anatomical feature using a first portion of the image around the point and using the position of the point relative to the image, and if the class into which the point is classified comprises sub-classes corresponding to sub-features of the anatomical feature, classify the point into a sub-class of the class using a second portion of the image around the point.

The processor may be for use in processing image data relating to the pelvic floor of a subject. The image data may be image data relating to the pelvic floor of the subject. The anatomical feature or sub-feature can be a feature or sub-feature usable for assessing the pelvic floor of the subject. The measurement can be a measurement usable for assessing the pelvic floor of the subject. It will be appreciated that examples herein are described in relation to processing image date relating to the pelvic floor or to measurement usable for assessing the pelvic floor, however, the proposed technical solution could be equally applied to other measurements or clinical applications, especially where more than one anatomical feature or sub-features (e.g. landmarks) are to be indicated or identified for measurements. For example, it can be applied to fetal scanning, where measurments can include any one or more of biparietal diameter, head circumference, abdominal circumference, femoral diaphsisi length as well as gestational age, fetal weight estimated based on one or more of the aforementioned measurements, and the corresponding anatomical features or sub-features can include any one or more of outer edge of the proximal skull, inner edge of the distal skull, ends of the femoral shaft along the long axis, the junction of the umbilical vein, poral sinums and fetal stomach, etc.

The image data may be any one of an ultrasound image and megnetic resonance image. The image data may be image data relating to a plane of a subject. The plane may be a sagittal plane, a coronal plane, an axial plane, (or any off-axis plane) for example. The sagittal plane may be a mid-sagittal plane. It will be appreciated that examples herein are described in relation to the mid-sagittal plane, however, the proposed technical solution could be applied to any planes which are used in the analysis of a pelvic floor of a subject. Furthermore, while the examples herein are described in relation to ultrasound imaging systems, it will be appreciated that the proposed technical solution herein may be equally applicable for magnetic resonance imaging (MRI) and therefore MRI systems may also embody the proposed technical solution herein. The methods herein may be equally applicable for images acquired by other existing or future developed imaging technology as well, such as computer tomographic (CT) imaging.

Advantageously, the proposed solution may automatically classify points into classes relating to a class or a sub-class, thereby reducing the requirement for user input and increasing the speed at which a points required for measurement are collected. Therefore, the time taken to process image data for diagnosis may be reduced.

The proposed solution may further comprise performing a measurement relating to the anatomical feature or sub feature corresponding to the class or sub-class into which the point is classified, and wherein the measurement is performed using the position of the point in the image. The method may further comprise classifying a plurality of points into classes and sub-classes, and performing the measurement once points have been identified as belonging to classes and/or sub-classes corresponding to all the anatomical features and/or sub-features required for the measurement to be performed.

According to a further aspect, there is provided an ultrasound imaging system comprising the apparatus as described herein and an ultrasound transducer with which to obtain image data relating to the image.

According to a further aspect there is provided a computer implemented method for use in processing image data relating to a subject, the method comprising: responsive to receiving input indicating a point in the an image corresponding to an anatomical feature or sub-feature, determining if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and if sufficient points to perform a measurement have been received, performing the measurement.

According to a further aspect there is provided a computer program product comprising computer readable medium, the computer readable medium having computer readable code embodied therein, the computer readable code being configured such that, on execution by a suitable computer or processor, the computer or processor is caused to perform the methods described herein.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 is an apparatus according to an example;
Fig. 2 is an ultrasound system according to an example;
Fig. 3 is a method according to an example;
Fig. 4 is an ultrasound image with indicated points and measurements;
Fig. 5 is a flow chart of a method according to an example;
Fig. 6 illustrates a process of receiving input points in ultrasound images;
Fig. 7 is an example of an ultrasound image of a mid-sagittal plane of a subject with markings indicating the location of anatomical features or sub-features and corresponding measurements.

### DETAILED DESCRIPTION OF EMBODIMENTS

In the standard practice of pelvic floor ultrasound, there are five steps in image acquisition and analysis including (the order of the steps may change in clinical practice):
1. a steady image of the mid-sagittal plane when a subject is at rest for assessing the baseline pelvic organ positions
2. a dynamic 2D image of the mid-sagittal plane when the subject is performing the Valsalva maneuver for assessing pelvic organ descent
3. a dynamic 3D image of the mid-sagittal plane when the subject is performing the Valsalva maneuver for hiatus reconstruction and assessment
4. a dynamic 3D image of the mid-sagittal plane when the subject is performing levator contraction for assessing levator ani
5. a dynamic 3D image of the coronal plane when the subject is performing levator contraction for assessing the anal sphincter muscles.

The current methods available for assessing pelvic floor ultrasound images may involve a clinician identifying relevant points corresponding to landmarks in an ultrasound image which can be used in order to perform a measurement. The clinician may click on the identified points and indicate the points that have been identified to an interface, along with a desired measurement, so that a computing device can then make the calculation or perform the measurement using the points.

Table 1 below illustrates the imaging steps and corresponding measurements to be performed for the each of the five imaging steps outlined above. Table 1 outlines the imaging step, the measurement along with the measurement type, and the points required to take a measurement. Table 1 also shows calculations that can be performed using the measurements. For example, for the first two imaging steps (of the mid-sagittal plane at rest and when the subject is performing the Valsalva maneuver), 11 measurements are performed in the first step, and 6 measurements are performed in the second step as shown in the table below. As can be seen from this table, in the same image, the same points may be used for different measurement (as an example, it can be seen that the symphysis pubis (SP) (e.g. a horizontal line through the SP) is used for measurement of the bladder to the SP, the uterus to the SP, the rectal ampulla to the SP, and so on, in the first imaging step). In current systems, a user may be required to identify a point corresponding to the SP (e.g. indicate a point on a horizontal line through the SP) each time a new measurement is to be performed.

**Table 1**

| **Imaging Step** | **Measurement Type** | **Measurement** | **Points** | **Calculation** |
|---|---|---|---|---|
| 1. Midsagittal plane at rest | Vertical Distance | Bladder to SP (BSD_r) | Bladder neck, SP (symphysis pubis) | |
| | Vertical Distance | Uterus to SP | Uterus neck, SP | |
| | Vertical Distance | Rectal ampulla to SP | Rectal ampulla, SP | |
| | Angle | Urethral tilt angle (UTA_r) | Bladder neck, urethral distal end | |
| | Angle | Retrovesical angle | Bladder neck, urethral distal end, point on bladder wall | |
| | Angle | Anorectal angle | Rectal ampulla, point on anal canal, point on rectum | |
| | Distance | Bladder long axis | Bladder long axis end 1 & 2 | Residual Bladder Volume (ml) = Bladder long axis (cm) * Bladder short axis (cm) * 5.6 |
| | Distance | Bladder short axis | Bladder short axis end 1 & 2 | |
| | Distance | Detrusor wall thickness_1 | Detrusor wall measurement line 1 end 1 & 2 | Detrusor wall thickness = mean of detrusor wall thickness_1 to 3 |
| | Distance | Detrusor wall thickness_2 | Detrusor wall measurement line 2 end 1 & 2 | |
| | Distance | Detrusor wall thickness_3 | Detrusor wall measurement line 3 end 1 & 2 | |
| 2. Midsagittal plane under Valsalva | Vertical Distance | Bladder to SP (BSD_v) | Bladder neck, SP | Bladder neck descent = BSD_r- BSD_v |
| | Vertical Distance | Uterus to SP | Uterus neck, SP | |
| | Vertical Distance | Rectal ampulla to SP | Rectal ampulla, SP | |
| | Angle | Urethral tilt angle (UTA_v) | Bladder neck, urethral distal end | Urethral rotation angle = UTA_r- UTA_v |
| | Angle | Retrovesical angle | Bladder neck, urethral distal end, point on bladder wall | |
| | Angle | Anorectal angle | Rectal ampulla, point on anal canal, point on rectum | |
| 3. Levator hiatus under Valsalva | Area | Hiatus area | | |
| | Distance | Hiatus long axis | | |
| | Distance | Hiatus short axis | | |
| 4. Levator ani under contraction | Distance | Levator ani thickness_1 | | Levator ani thickness = mean of levator ani thickness_1 to 3 |
| | Distance | Levator ani thickness_2 | | |
| | Distance | Levator ani thickness_3 | | |
| | Distance | Levator-urethral gap_left | | |
| | Distance | Levator-urethral gap_right | | |
| 5. Anal Sphincter under contraction | Angle | Anal sphincter defect angle_1 | | |
| | Angle | Anal sphincter defect angle_2 | | |
| | Angle | Anal sphincter defect angle_3 | | |
| | Angle | Anal sphincter defect angle_4 | | |
| | Angle | Anal sphincter defect angle_5 | | |

Imaging steps and corresponding measurements for analysis of the pelvic floor

In order to reduce the length of time required to obtain the required measurements and provide a more efficient system, methods described herein are provided in which when a user indicates a point in an image, a measurement can be performed once sufficient points to perform the measurement have been obtained. Furthermore, a point relating to an anatomical features or sub feature can be re-used for different measurements, so it is not necessary for a clinician to re-click on a point. The points may be stored so that it is not necessary to repeatedly indicate the same anatomical feature or sub feature in an image.

Furthermore, methods are described herein where the anatomical feature or sub feature corresponding to the point can be automatically classified, and when all points corresponding to a measurement have been classified, the measurement may be performed using the points.

As described above, the apparatus and methods described herein allow for improved efficiency of processing of image data relating to a pelvic floor of a subject.

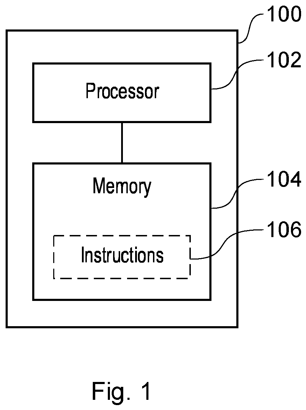

Turning now to Fig. 1, which shows an apparatus 100 for use in processing image data relating to a pelvic floor of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

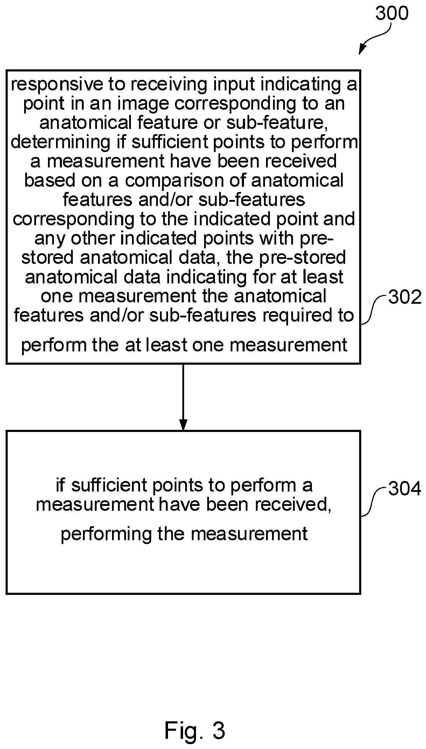

Generally, the apparatus may be adapted or configured to perform any of the embodiments of the methods described herein, such as the method 300 as described below with respect to Fig. 3.

The apparatus comprises a memory 104 comprising instruction data representing a set of instructions 106 and a processor 102 (e.g. processing circuitry or logic) configured to communicate with the memory and to execute the set of instructions 106. Briefly, the set of instructions 106, when executed by the processor 102, cause the processor 102 to, responsive to receiving input indicating a point in the an image corresponding to an anatomical feature or sub-feature, determine if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and if sufficient points to perform a measurement have been received, perform the measurement.

As described above, current methods for assessing pelvic floor ultrasound images may require a user to indicate the same points in an image when the same point is required for a different measurement. Furthermore, current methods may require selection of a measurement name to enable a calculation across measurements, and there may be little flexibility in the order in which points are required to be indicated by the user or the measurements that are performed. The proposed apparatus herein advantageously improves the efficiency of processing of image data.

In more detail, the processor 102 may comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the sequence of ultrasound image data and/or any other information or data received, calculated or determined by the processor 102 of the apparatus 100 or from any interfaces, memories or devices that are external to the apparatus 100. The processor 102 may be configured to control the memory 104 to store the sequence of ultrasound image data and/or the any other information or data received, calculated or determined by the processor.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 may be comprised in an ultrasound system. For example, an ultrasound system may comprise the apparatus 100 described above, and further comprise a transducer with which to record the sequence of ultrasound image data.

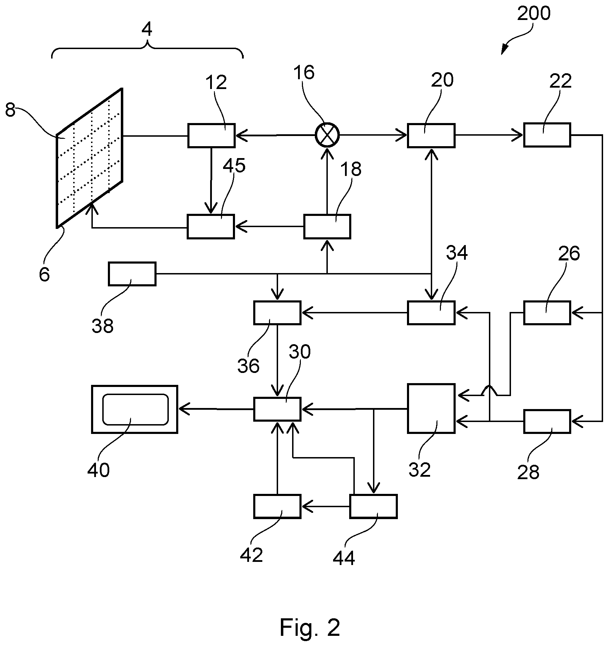

Fig. 2 shows an example ultrasound system 200. The ultrasound system 200 may comprise the apparatus 100 described above. In other embodiments, components of the ultrasound system 200 may be adapted to perform the method 300 described below.

The system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes.

The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, where instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38, such as the seed points for locating the surface of the muscle, as described in detail below.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The skilled person will appreciate that the detail provided above and the components illustrated in Fig. 2 are an example only and that an ultrasound system may have different components to those illustrated therein.

Turning now back to Fig. 1, and the functionality of the processor 102, as noted above, the processor 102 is caused to, responsive to receiving input indicating a point in an image corresponding to an anatomical feature or sub-feature, determine if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and if sufficient points to perform a measurement have been received, perform the measurement.

Turning to Fig. 3, there is a computer implemented method 300 for use in processing image data relating to a pelvic floor of a subject. Embodiments of the method 300 may be performed, for example by an apparatus such as the apparatus 200 described above.

Briefly, in a first step 302, the method 300 comprises, responsive to receiving input indicating a point in an image corresponding to an anatomical feature or sub-feature, determining if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement. In a second step 304 the method comprises, if sufficient points to perform a measurement have been received, performing the measurement.

The pre-stored anatomical data may be a data set which comprises a list of anatomical features and/or sub-features, where groups of features from the list may be usable, or required, to perform particular measurements. The method may therefore determine when all features and/or sub-features in a particular group corresponding to a measurement have been indicated. The data set may comprise some or all of the information illustrated in table 1. It will be appreciated that the same anatomical feature or sub-feature may be used to perform different measurements, e.g. the same anatomical feature or sub-feature may be used to perform more than one measurement. The determining may involve comparing any received points (e.g. any points that have been indicated by the user for the same image data to the pre-stored anatomical data to determine if the points which have been indicated can be used to perform a measurement). Thus, received points may be stored.

A point may be determined as corresponding to an anatomical feature or sub feature based on received input from a user indicating the anatomical feature or sub feature to which the point corresponds. For example, after indicating a point, a user may select a corresponding anatomical feature or sub feature, e.g. from a drop down menu or the like. Alternatively, the anatomical feature or sub-feature to which the point corresponds may be determined by classifying the point into a class corresponding to the anatomical feature or sub feature, for example using an algorithm.

The method may further comprise determining to which anatomical feature or sub feature the point corresponds by classifying the point into a class corresponding to an anatomical feature using a first portion of the image around the point and using the position of the point relative to the image, and if the class into which the point is classified comprises sub-classes corresponding to sub-features of the anatomical feature, classify the point into a sub-class of the class using a second portion of the image around the point.

The method may be for use in processing image data relating to a pelvic floor of a subject. In particular the image data may comprise a plane of a user, such as a sagittal plane (e.g. a mid-sagittal plane), a coronal plane, an axial plane, (or any off-axis plane) for example. The anatomical features or sub features may usable to assess the pelvic floor of a subject. Where the method is used for assessing pelvic floor, the anatomical feature may be at least one of: symphysis pubis, bladder region, uterus, anorectal region. A point may be determined as corresponding to one of these anatomical features. However, some of these anatomical features have sub-features which may also be usable for assessing the pelvic floor of a subject. Therefore, it may be advantageous to identify a sub-feature of an anatomical feature where an anatomical feature comprises a sub-feature. For example, for a bladder region, there are several points of interest, such as the bladder neck, the urethral distal end and the bladder wall. Therefore, it may be considered that the bladder region is a general anatomical feature (corresponding to a class) and the bladder neck and the bladder wall are sub-features (corresponding to sub-classes) of the bladder region. In an example, the user may identify the feature or sub-feature to which the point corresponds. In a further example, the method may classify the feature or sub-feature.

Where the method comprises classification of a point, where a point is determined as being within the bladder region in the method, the method may therefore go on to identify the point as corresponding to a further sub-feature of the bladder. The sub-anatomical features may be at least one of: bladder neck; urethral distal end; point on bladder wall; rectal ampulla; point on anal canal; point on rectum; uterus neck; first bladder long axis end; second bladder long axis end; first bladder short axis end; second bladder short axis end; first detrusor wall measurement line 1 end; second detrusor wall measurement line 1 end; first detrusor wall measurement line 2 end; second detrusor wall measurement line 2 end; first detrusor wall measurement line 3 end; second detrusor wall measurement line 3 end; urethra; posteria wall of bladder; detrusor wall upper border; detrusor wall lower border; first bladder side; second bladder side; third bladder side; fourth bladder side; anal canal, and so on.

It will be appreciated that the method is not limited to the detection or identification of these anatomical features or sub features, and may be applied to detect or identify any relevant features. These anatomical features or sub features may be usable in order for the method to perform measurements based on the position of the point or points in the image. The measurement may be a measurement usable for assessing the pelvic floor of a subject.

The received input may be input from a user interacting with an interface, and may be, for example, a click on a point in an image corresponding to an anatomical feature. It will be appreciated that the method is not limited herein to a two dimensional (2D) image or image data, and could instead be applied to three dimensional (3D) images or image data, or 4D images or image data. Thus, the first portion of the image around the point and/or the second portion of the image around the point may be an area or may be a volume. It will be further appreciated that while a user may interact with a 2D image, the 2D image may be a representation of a 3D (or 4D) image or image data, and therefore the point indicated in the 2D representation may be used to determine a volume around the point in a 3D image corresponding to the 2D representation.

The first portion of the image may be larger than the second portion of the image. In particular, the first portion of the image may comprise a larger area or a larger volume than the second portion of the image. For example, the first portion of the image may be used to determine the general location of the indicated point, such as in relation to an organ of a subject. The second portion of the image may be used to determine a more specific location of the indicated point, in particular in relation to a component of an organ. Therefore, the first portion may be larger to determine a general location of the point, and once the general location has been determined, the second portion may be smaller in order to more specifically locate the point. It will be appreciated that a larger region will have more distinctive characteristics than a smaller region, and therefore classification of a larger region may be performed more quickly and more accurately than a smaller region. Furthermore, by first classifying the point into a region before classifying the point into a sub-region, the accuracy and speed of the second classification may be improved, as there will be a reduced number of options for the sub-region based on the region into which the point has been classified compared to the number of sub-regions that may be present in the whole image (e.g. corresponding to different anatomical features). The size of the first region may be a first predetermined size and the size of the second region may be a second predetermined size. The sizes may be proportionate to the overall size of the image, or may be pre-set sizes . In an example, the first potion may be large enough to encompass an anatomical feature, and may be in the region of 300-500 square pixels for an image of size 1024*768 pixels. For the second portion, the size may depend on the classification result relating to the first portion (e.g. the size of the second portion may depend on the anatomical features that is the result of the classification of the first portion). The second portion may be in the region of 100-200 square pixels for an image of size 1024*768 pixels.

The classification may be performed using a classification algorithm, or a classification model, which has been trained using portions of training images around points corresponding to at least one of the anatomical features and the anatomical sub-features, or has been developed based on an image feature extraction method (e.g. using shape, texture and so on). Any appropriate image processing methods or deep learning methods may be used in the classification of points. For example, using training data of portions of an image labelled as the relevant anatomical features or sub-features, the classification model may be trained to classify an anatomical feature or sub feature from input of the first portion of the image and/or the second portion of the image. The classification model may also take as input the position of the point relative to the image to assist in the classification. It will be appreciated that any appropriate classification model, such as a neural network, machine learning model, deep learning neural network, image feature extraction and regression, and so on, may be used in the methods described herein.

The skilled person will be familiar with neural networks, but in brief, neural networks are a type of supervised machine learning model that can be trained to predict a desired output for given input data. Neural networks are trained by providing training data comprising example input data and the corresponding "correct" or ground truth outcome that is desired. Neural networks comprise a plurality of layers of neurons, each neuron representing a mathematical operation that is applied to the input data. The output of each layer in the neural network is fed into the next layer to produce an output. For each piece of training data, weights associated with the neurons are adjusted until the optimal weightings are found that produce predictions for the training examples that reflect the corresponding ground truths.

The skilled person will appreciate that generally the trained model may comprise any model that may be trained to take as input an image product and output a classification for one or more features in an image.

The point may be stored with a label indicating the anatomical feature or sub feature to which the point corresponds. For example, the point may be stored with a label indicating the class or sub class into which it has been classified. The point may be displayed on a display showing the image or a representation of the image. In this way, a user may be able to determine which points have been previously obtained, and avoid repeating the same input (e.g. clicks on the same point). For particular points, additional features may be determined and/or displayed on the display. For example, if the point is identified by a user as, or is classified into a class, corresponding to the symphysis pubis (SP), the method may cause a horizontal line through the point to be determined, and may displayed the line on a display showing the image.

The method may comprise performing a measurement. For example, a measurement is performed if sufficient points have been received to perform the measurement. In particular, the measurement may be performed using the position of the point in the image. For example, several anatomical features may be required to perform a measurement. Each time input is received indicating a point in the image, the point may also be indicated by a user as corresponding to an anatomical feature or sub feature, and when enough anatomical features have been identified that a measurement can be performed, the method may further comprise performing the measurement. For pelvic floor images, the measurement may be at least one of: bladder to symphysis pubis; uterus to symphysis pubis, rectal ampulla to symphysis pubis; urethral tilt angle; retrovesical angle; anorectal angle; bladder long axis; bladder short axis; first detrusor wall thickness; second detrusor wall thickness; third detrusor wall thickness; hiatus area; hiatus long axis; hiatus short axis; first levator ani thickness; second levator ani thickness; third levator ani thickness; left levator-urethral gap; right levator urethral gap; first anal sphincter defect angle; second anal sphincter defect angle; third anal sphincter defect angle; fourth anal sphincter defect angle; fifth anal sphincter defect angle. It will be appreciated that any relevant measurements may be taken using located anatomical or sub anatomical features in an image of a subject.

The method may further comprise performing a measurement relating to the anatomical feature or sub feature corresponding to the class or sub-class into which the point is classified, and wherein the measurement is performed using the position of the point in the image. The method may further comprise classifying a plurality of points into classes and sub-classes, and perform the measurement once points have been identified as belonging to classes and/or sub-classes corresponding to all the anatomical features and/or sub-features required for the measurement to be performed. For example, several anatomical features may be required to perform a measurement. Each time input is received indicating a point in the image, the point may be classed as belonging to a particular class or sub class corresponding to an anatomical feature or sub feature, and when enough anatomical features have been identified that mean a measurement can be performed, the method may further comprise performing the measurement. For pelvic floor images, the measurement may be at least one of: bladder to symphysis pubis; uterus to symphysis pubis, rectal ampulla to symphysis pubis; urethral tilt angle; retrovesical angle; anorectal angle; bladder long axis; bladder short axis; first detrusor wall thickness; second detrusor wall thickness; third detrusor wall thickness; hiatus area; hiatus long axis; hiatus short axis; first levator ani thickness; second levator ani thickness; third levator ani thickness; left levator-urethral gap; right levator urethral gap; first anal sphincter defect angle; second anal sphincter defect angle; third anal sphincter defect angle; fourth anal sphincter defect angle; fifth anal sphincter defect angle. It will be appreciated that any relevant measurements may be taken using located anatomical or sub anatomical features in an image of a subject.

Once a measurement has been performed, the result of the measurement may be stored. Once measurements have been performed corresponding to all the measurements required to make a further measurement, the further measurement may be performed. For example, a calculation may be performed using at least one measurement. The measurements and/or further measurements may be stored.

The identified anatomical features (e.g. the class or sub class the point is classified into, or is identified by a user) may be stored in a memory or register. It will be appreciated that it is not necessary for a user to repeatedly indicate an anatomical feature that is usable to perform more than one measurement. For example, once an anatomical feature has been located, it may be used in any measurement that requires that anatomical feature. This is advantageous as it does not require a user repeatedly inputting the same point, and therefore improves efficiency of processing image data.

The classification of the point may be further based on the classification of a preceding point. For example, if the method has classified a point which has been previously indicated, it may use this classification to determine the classification of a current point. In particular, a user indicating a sequence of points may do so based on a predetermined sequence. For example, to measure the retrovesical angle, a user will typically click on the urethra, followed by the bladder neck, and then the posterior wall of the bladder. Therefore, the classification may expect points to follow a particular sequence of input of points. The method may therefore use the location of the point in an order of input of points relative to a predicted sequence of input of points in order to classify a current point. Furthermore, the position of the point in the image relative to a previously classified point may be used to determine the classification of a current point. For example, two components of the same organ may typically have a similar relative position to one another even in different subjects or images. Therefore, where a currently indicated point is positioned relative to a previously indicated point at a location where an anatomical feature is expected based on the anatomical feature corresponding to the classification of the previously classified point, the classification may also be based on whether the anatomical feature is expected to be at the location.

The confidence of the classification of a previously classified point may be updated based on the classification of the point. For example, if a current point is classified into the same class (or sub class) as a previously classified point, the confidence of the previously classified point may be reduced. If a current point is classified into a different class (or sub class) to any previously classified point, the confidence of the previously classified point may be increased.

The confidence of the classification of a previously classified point may be updated based on the position of the point in the image relative to the previously classified point. For example, where a previously classified point (e.g. corresponding to an anatomical feature) is expected to be located relative to another point corresponding to another anatomical feature, classification of a current point may be used to determine if the previously classified point has likely been correctly identified. If it is determined that the confidence of the previously classified point has likely been correctly identified based on the classification and location of the current point, the confidence of the classification of the previous point may be increased. Similarly, if it is determined that the confidence of the previously classified point has likely been incorrectly identified based on the classification and location of the current point, the confidence of the classification of the previous point may be decreased.

The method may further comprise, if the point is classified into the same class, or the same sub-class where the class is separable into sub-classes, as a previously classified point, requesting input indicating which classification should be used for at least one of the point and the previously classified point. The method may further comprise, responsive to receiving an indication of a classification to be used for at least one of the point or the previously classified point, updating the classification of at least one of the point or the previously classified point based on the received indication. Thus, the accuracy of classification can be improved by the confirmation or alteration of a classification.

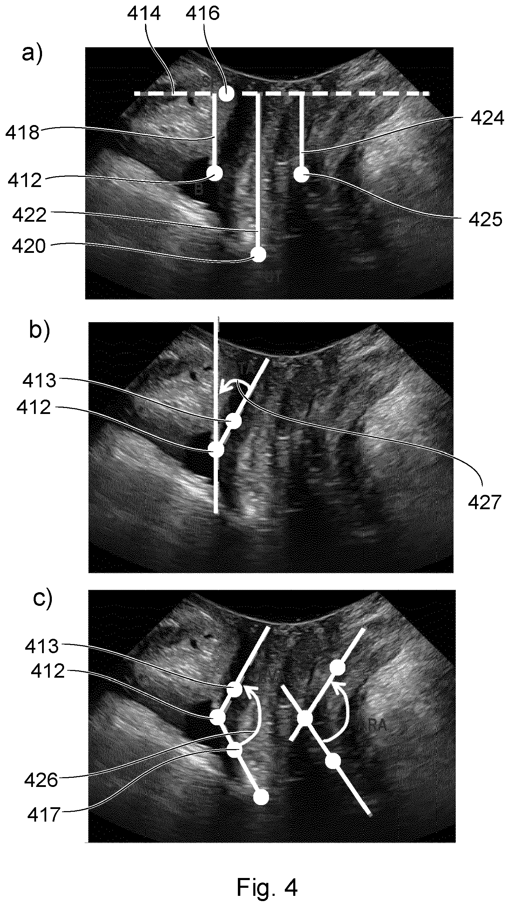

In an example, ultrasound images may be obtained by the systems described above. For example, a user can use the ultrasound system with either a linear probe or a volume probe to acquire 2D images of the mid-sagittal plane at the rest state of a subject. The user may then indicate a point in the image by clicking on a point in the image. In an example of pelvic floor assessment, the click may be any point of the 18 points needed as the input to perform one or more of the 11 measurements (the required points and measurements are outlined in table 1). The method may then comprise determining which of the 18 landmarks (anatomical features or sub features) the user has clicked. As discussed above, the method may use a two-step classification using two differently sized portions of the images around the clicked points. In addition, the position of the clicked point within the image may also be used for the classification. Alternatively, a user may indicate which of the 18 landmarks the user has clicked. Fig. 4 illustrates examples of ultrasound images relating to a pelvic floor of a subject (in particular a mid sagittal plane) where points corresponding to anatomical features or sub features are indicated by dots (points). In this example, points corresponding to SP, bladder neck, uterus, and rectal ampulla have been identified. These points may have been indicated (e.g. by a user). As is illustrated in this Figure, these points may be used in order to perform measurements.

Fig. 4(a) illustrates an example of an ultrasound image. In an existing system, to measure the distance from the bladder neck (B) 412 to a horizontal reference line 414 through the symphysis pubis (SP) 416, the user is required to identify and click on two points to create the horizontal reference line 414 through the SP (the horizontal reference line through the SP is an important reference line for judging organ prolapse), and to identify and click on two further points to create a vertical line to take the distance measurement from the bladder neck 412 to the horizontal reference line 414 through the SP 416 - one on the bladder neck 412 and one on the horizontal reference line 414. The user is also required to indicate which points have been clicked to a computing device, and indicate which measurement is to be performed. A similar process may be used to determine the distance 422 from the uterus (UT) 420 to the horizontal reference line 414, and the distance 424 from the rectal ampulla (RA) 425 to the horizontal reference line 414.

However, the methods described herein may instead automatically perform a measurement once sufficient anatomical features or sub features have been identified for that measurement to be performed. For example, there may be provided a pre-stored set of anatomical features or sub-features, where groups of features may be usable in order to perform a measurement. Therefore, by comparison of the identified features or sub features with the pre-stored set of features, it can be determined if sufficient features or sub features have been identified/indicated to perform a measurement, and the measurement may be performed once the features required for a measurement have been identified. Furthermore, the method may automatically classify a point using a portion of the image around the point.

Once a point has been identified, a measurement may be performed and may be displayed on the image currently being viewed by a user. As an example, once the SP has been identified, a horizontal line passing through the SP may be determined. Once a point corresponding to one of the bladder neck, the uterus, or the rectal ampulla, has been identified the method may comprise automatically performing the measurements outlined above between each of these features and the horizontal line passing through the SP. The distance between one of the bladder neck, the uterus or the rectal ampulla, and the SP, may be shown on the image.

There are two further measurements including the urethral tilt angle 427 (shown in Fig. 4(b) and the retrovesical angle 426 (shown in Fig. 4(c)) that also use the bladder neck landmark 412 which was obtained in relation to Fig. 4(a). In order to obtain these further measurements, in current methods, it may be necessary for a user to click again on the same anatomical landmark to take a measurement, which is the bladder neck 412 in this case, in addition to the urethral distal end 413 for the urethral tilt angle 427, and the urethral distal end 413 and a point on the bladder wall 417 for the retrovesical angle 426. However, using the methods described herein, once an anatomical feature has been identified, there is no need for a user to re-find the same feature to perform a different measurement. In this example, that means that once the bladder neck 412 and urethral distal end 413 have been identified, the urethral tilt angle 427 may be determined, and only one further click to identify a point on the bladder wall is required in order that the points required for the retrovesical angle measurement to be performed are obtained.

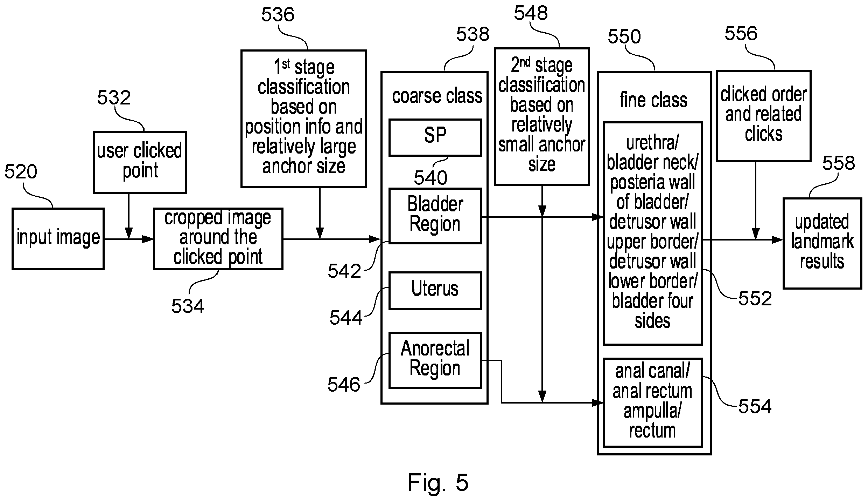

Fig 5 illustrates a flow chart of the method according to an example in which classification is performed. As can be seen in this Figure, the method may comprise receiving an image 530, and receiving input indicating a point in the image 532, for example, a point clicked by a user. A first portion of the image may be obtained, for example by cropping the image around the point 534. A first classification stage may then classify the point into a class corresponding to an anatomical feature using a first portion of the image around the point and using the position of the point relative to the image 536, where the first portion of the image is relatively large. The point may therefore be classified into a coarse class 538. For example, where the image is related to pelvic floor assessment, the coarse class may be divided into the four classes based on the distinctive location of the anatomical features relating to the class in the image and the distinctive echo presentation in these regions. In particular, the classes in this example are SP 540, bladder region 542, uterus 544 and anorectal region 546. Once the region has been narrowed down, a second classification may be performed 548 for anatomical features which may be divisible into sub features. In this example, the bladder region and the anorectal region are separable into a number of smaller anatomical sub-features. In particular, the bladder region may be divisible into sub-classes of urethra, bladder neck, posteria wall of bladder, detrusor wall upper border, detrusor wall lower border, four sides of the bladder 552. The anorectal region may be divisible into sub regions of the anal canal, analrectum ampulla, and rectum 554. A second portion, e.g. a smaller ROI/detection anchor size, may be used to separate the landmarks that are crowded in nearby locations by classifying the point using the second portion. Once a class or sub-class has been determined for a point, the click order and related clicks 556 may be used in order to improve the confidence of a classification. For example, it may be assumed that the user will click the landmarks (e.g. the anatomical features or sub-features) in a rational order, where this information can then be used to update the classification results and boost the confidence of the classification. For example, to measure the retrovesical angle, a user will typically click on the urethra, followed by the bladder neck, and then the posterior wall of the bladder. The click on the posterior wall of the bladder may be mistaken as one of the points on the bladder long axis for measuring the residual bladder volume. If the click order and prior click classification information is used, the method may reduce the chance of mistaking similar and close-by landmarks. The landmark result may therefore be updated 558.

Once the classification has been performed, the method may further comprise enabling the user to correct the classification result if needed. The user may use a user interface such as a control panel or a mouse to access a drop-down menu of classification results listed in the order of classification likelihood. The classified result of the point may be registered in an internal processor/memory. If the user clicks on a point that highly coincidence with a prior point and is therefore classified into the same class or sub class, another function may be triggered to enable the user to confirm whether the prior classified landmark needs to be updated.

A processor may be continuously searching for identified features or sub features so that once all the required inputs for a measurement are completed, the measurement will be automatically computed and displayed on the image. If insufficient input is provided, then the processor may wait for the next input until enough features or sub features are identified for a measurement.

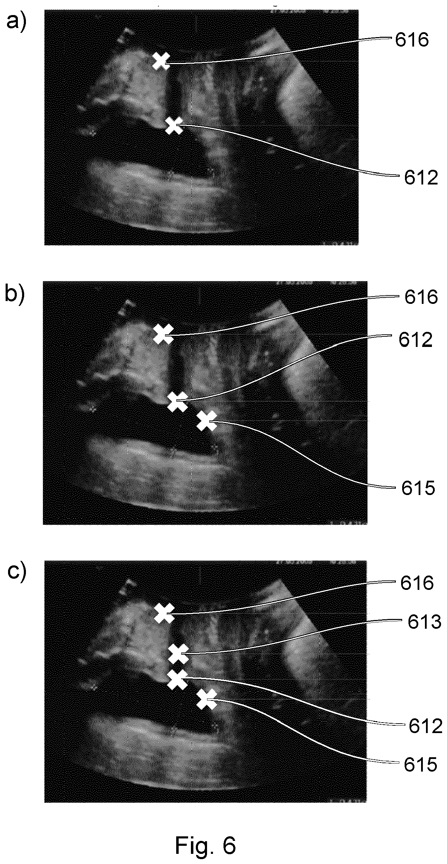

This process is illustrated in the Figure 6. For example, in Fig. 6(a) input corresponding to clicks on points in the image is received (shown as crosses in the images). Using the methods described herein, each point is either indicated by a user as corresponding to a feature or sub feature, or classified. In this example, the two points marked by an X in the image correspond to the SP 616 and the bladder neck 612. The positions corresponding to these features are stored along with the features. The two points of SP and bladder neck are used to determine the first bladder distance. A further point of the bladder posterior wall 615 is identified based on a point clicked by a user illustrated in Fig. 6(b). However, these three points are not sufficient to perform any further measurement, and therefore no further measurement is performed. Fig. 6(c) illustrates a further point clicked by a user which is identified as the urethra 613. With this additionally identified point, it becomes possible to perform measurements of a urethral incline angle as well as a retrovesical angle using the identified points. These two further measurements are therefore performed using the points.

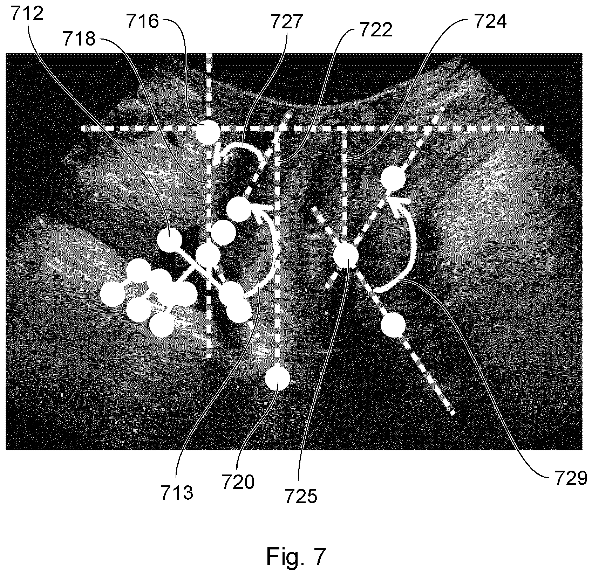

Fig. 7 illustrates an example of an ultrasound image of a mid-sagittal plane of a subject with markings indicating the location of anatomical features or sub-features which may be identified using the methods described herein. Furthermore, this Figure illustrates an example of measurements which may be performed using the located anatomical features once the relevant anatomical features required to perform a measurement have been located.

In particular, this Figure illustrates anatomical features such as the SP: symphysis pubis 716, B: bladder 712, UT: uterus 720, RA: rectal ampulla 725. Further features and sub features are illustrated as the additional dots in the image (corresponding to the eighteen points required to make the eleven measurements in relation to an image of the mid-sagittal plane at rest). Measurements are illustrated as dotted lines. Measurements shown in this Figure include the vertical distance from bladder/uterus/rectal ampulla to symphysis pubis 718, 722, 724, angle measurements including UTA: urethra tilt angle 727, RVA: retrovesical angle 713, ARA: anorectal angle 729, the bladder long and short axes, and 3 bladder wall thickness measurements.

The method may further comprise performing a further measurement using a previously determined measurement. For example, once a measurement of the long and short axes for the bladder has been performed, the residual bladder volume may be calculated as being Residual Bladder Volume (ml) = Bladder long axis (cm) * Bladder short axis (cm) * 5.6.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for use in processing image data relating to a subject, the apparatus comprising:
a memory (104) comprising instruction data representing a set of instructions; and
a processor (102) configured to communicate with the memory and to execute the set of instructions (106), chacterized in that the set of instructions, when executed by the processor, cause the processor to:
responsive to receiving input indicating a point (612, 615, 616, 613) in an image corresponding to an anatomical feature or sub-feature of the subject,
determine (302) if sufficient points to perform a measurement (727, 713) have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and
if sufficient points to perform a measurement have been received, perform (304) the measurement.

2. The apparatus as claimed in claim 1, wherein the processor (102) is further caused to determine to which anatomical feature or sub-feature the point corresponds based on received input from a user indicating the anatomical feature or sub-feature to which the point corresponds, or by classifying the point into a class corresponding to the anatomical feature or sub-feature .

3. The apparatus as claimed in claim 1 or 2, wherein the processor (102) is further caused to determine to which anatomical feature or sub-feature the point corresponds by classifying the point into a class corresponding to an anatomical feature using a first portion of the image around the point and using the position of the point relative to the image, and
if the class into which the point is classified comprises sub-classes corresponding to sub-features of the anatomical feature, classify the point into a sub-class of the class using a second portion of the image around the point.

4. The apparatus as claimed in claim 3, wherein the classification of the point is further based on at least one of: the classification of a preceding point; the position of the point in the image relative to a previously classified point; the location of the point in an order of input of points relative to a predicted sequence of input of points.

5. The apparatus as claimed in any of claims 3 or 4, wherein confidence of the classification of a previously classified point is updated based on at least one of: the classification of the point; the position of the point in the image relative to the previously classified point.

6. The apparatus as claimed in any of claims 3 to 5, wherein the processor (102) is further configured to:
if the point is classified into the same class, or the same sub-class where the class is separable into sub-classes, as a previously classified point, request input indicating which classification should be used for at least one of the point and the previously classified point; and
responsive to receiving an indication of a classification to be used for at least one of the point or the previously classified point, updating the classification of at least one of the point or the previously classified point based on the received indication.

7. The apparatus as claimed in any of claims 3 to 6, wherein the classification is performed using a classification algorithm which has been trained using portions of training images around points corresponding to at least one of the anatomical features and the anatomical sub-features.

8. The apparatus as claimed in any of claim 3 to 7, wherein the first portion of the image is larger than the second portion of the image.

9. The apparatus as claimed in any preceding claim , wherein the measurement is performed using the position of the point in the image.

10. The apparatus as claimed in any preceding claim, wherein the processor (102) is further configured to at least one of: store the point with a label corresponding to the anatomical feature or sub-feature; cause the point to be displayed on a display showing the image; when the point corresponds to the symphysis pubis, cause a horizontal line through the point to be displayed on a display showing the image.

11. The apparatus as claimed in any preceding claim, wherein at least one of: the anatomical feature or sub-feature is a feature or sub-feature usable for assessing a pelvic floor of the subject; the measurement is a measurement usable for assessing a pelvic floor of the subject.

12. The apparatus as claimed in any preceding claim, wherein the image data is any one of an ultrasound image and magnetic resonance image of the subject.

13. An ultrasound imaging system (200) comprising the apparatus (100) of any one of the preceding claims and an ultrasound transducer (4, 6) with which to obtain image data relating to the image.

14. A computer implemented method (300) for use in processing image data relating to a subject, the method comprising:
responsive to receiving input indicating a point in an image corresponding to an anatomical feature or sub-feature of the subject,
determining (302) if sufficient points to perform a measurement have been received based on a comparison of anatomical features and/or sub-features corresponding to the indicated point and any other indicated points with pre-stored anatomical data, the pre-stored anatomical data indicating for at least one measurement the anatomical features and/or sub-features required to perform the at least one measurement; and
if sufficient points to perform a measurement have been received, performing (304) the measurement.

15. A computer program product comprising computer readable medium the computer readable medium having computer readable code embodied therein, the computer readable code being configured such that, on execution by a suitable computer or processor, the computer or processor is caused to perform the method as claimed in claim 14.

## Patentansprüche

1. Einrichtung zur Verwendung bei der Verarbeitung von Bilddaten, die sich auf ein Subjekt beziehen, wobei die Einrichtung umfasst:
einen Speicher (104), der Anweisungsdaten umfasst, die einen Satz von Anweisungen darstellen; und
einen Prozessor (102), der dazu konfiguriert ist, mit dem Speicher zu kommunizieren und den Satz von Anweisungen (106) auszuführen, **dadurch gekennzeichnet, dass** der Satz von Anweisungen, wenn er von dem Prozessor ausgeführt wird, den Prozessor zu Folgendem veranlasst:
als Reaktion auf Empfangen einer Eingabe, die einen Punkt (612, 615, 616, 613) in einem Bild angibt, der einem anatomischen Merkmal oder Untermerkmal des Subjekts entspricht,
zu bestimmen (302), ob genügend Punkte empfangen worden sind, um eine Messung (727, 713) durchzuführen, basierend auf einem Vergleich von anatomischen Merkmalen und/oder Untermerkmalen, die dem angegebenen Punkt und allen anderen angegebenen Punkten mit vorgespeicherten anatomischen Daten entsprechen, wobei die vorgespeicherten anatomischen Daten für zumindest eine Messung die anatomischen Merkmale und/oder Untermerkmale angeben, die erforderlich sind, um die zumindest eine Messung durchzuführen; und
wenn genügend Punkte empfangen wurden, um eine Messung durchzuführen, die Messung durchzuführen (304).

2. Einrichtung nach Anspruch 1, wobei der Prozessor (102) weiter veranlasst wird, zu bestimmen, welchem anatomischen Merkmal oder Untermerkmal der Punkt entspricht, basierend auf einer empfangenen Eingabe von einem Benutzer, die das anatomische Merkmal oder Untermerkmal angibt, dem der Punkt entspricht, oder durch Klassifizieren des Punkts in eine Klasse, die dem anatomischen Merkmal oder Untermerkmal entspricht.

3. Einrichtung nach Anspruch 1 oder 2, wobei der Prozessor (102) weiter veranlasst wird, zu bestimmen, welchem anatomischen Merkmal oder Untermerkmal der Punkt entspricht, durch Klassifizieren des Punkts in eine Klasse, die einem anatomischen Merkmal entspricht, unter Verwendung eines ersten Abschnitts des Bilds rund um den Punkt und Verwendung der Position des Punkts in Bezug zu dem Bild, und
wenn die Klasse, in die der Punkt eingeordnet wird, Unterklassen umfasst, die Untermerkmalen des anatomischen Merkmals entsprechen, den Punkt unter Verwendung eines zweiten Abschnitts des Bilds rund um den Punkt in eine Unterklasse der Klasse zu klassifizieren.

4. Einrichtung nach Anspruch 3, wobei die Klassifikation des Punkts weiter auf zumindest einem basiert von: der Klassifikation eines vorhergehenden Punkts; der Position des Punkts in dem Bild in Bezug zu einem zuvor klassifizierten Punkt; der Stelle des Punkts in einer Reihenfolge der Punkteingabe in Bezug zu einer vorhergesagten Eingabesequenz von Punkten.

5. Einrichtung nach einem der Ansprüche 3 oder 4, wobei die Zuverlässigkeit der Klassifikation eines zuvor klassifizierten Punkts basierend auf zumindest einem aktualisiert wird von: der Klassifikation des Punkts; der Position des Punkts in dem Bild in Bezug zu einem zuvor klassifizierten Punkt.

6. Einrichtung nach einem der Ansprüche 3 bis 5, wobei der Prozessor (102) weiter konfiguriert ist, um:
wenn der Punkt in dieselbe Klasse oder, wo die Klasse in Unterklassen teilbar ist, in dieselbe Unterklasse wie ein zuvor klassifizierter Punkt klassifiziert wird, Eingabe anzufordern, die angibt, welche Klassifikation für zumindest einen von dem Punkt und dem zuvor klassifizierten Punkt verwendet werden sollte; und
als Reaktion auf Empfangen einer Angabe einer Klassifikation, die für zumindest einen von dem Punkt oder dem zuvor klassifizierten Punkt verwendet werden sollte, Aktualisieren der Klassifikation von zumindest einem von dem Punkt oder dem zuvor klassifizierten Punkt basierend auf der empfangenen Angabe.

7. Einrichtung nach einem der Ansprüche 3 bis 6, wobei die Klassifikation unter Verwendung eines Klassifikationsalgorithmus durchgeführt wird, der unter Verwendung von Abschnitten von Trainingsbildern rund um Punkte trainiert worden ist, die zumindest einem der anatomischen Merkmale und der anatomischen Untermerkmale entsprechen.

8. Einrichtung nach einem von Anspruch 3 bis 7, wobei der erste Abschnitt des Bilds größer ist als der zweite Abschnitt des Bilds.

9. Einrichtung nach einem vorstehenden Anspruch, wobei die Messung unter Verwendung der Position des Punkts in dem Bild durchgeführt wird.

10. Einrichtung nach einem vorstehenden Anspruch, wobei der Prozessor (102) weiter für zumindest eines konfiguriert ist von: den Punkt mit einer Markierung zu speichern, die dem anatomischen Merkmal oder Untermerkmal entspricht; den Punkt zu veranlassen, auf einem Display angezeigt zu werden, welches das Bild zeigt; wenn der Punkt der Symphysis pubica entspricht, eine horizontale Linie durch den Punkt hindurch zu veranlassen, auf einem Display, welches das Bild zeigt, angezeigt zu werden.

11. Einrichtung nach einem vorstehenden Anspruch, wobei zumindest eines von: dem anatomischen Merkmal oder Untermerkmal ein Merkmal oder Untermerkmal ist, das zum Beurteilen eines Beckenbodens des Subjekts verwendbar ist; der Messung eine Messung ist, die zum Beurteilen eines Beckenbodens des Subjekts verwendbar ist.

12. Einrichtung nach einem vorstehenden Anspruch, wobei die Bilddaten eines von einem Ultraschallbild oder einem Magnetresonanzbild des Subjekts sind.

13. Ultraschallbildgebungssystem (200), umfassend die Einrichtung (100) nach einem der vorstehenden Ansprüche und einen Ultraschallwandler (4, 6), mit dem Bilddaten erhalten werden, die sich auf das Bild beziehen.

14. Computerimplementiertes Verfahren (300) zur Verwendung bei der Verarbeitung von Bilddaten, die sich auf ein Subjekt beziehen, wobei das Verfahren Folgendes umfasst:
als Reaktion auf Empfangen einer Eingabe, die einen Punkt in einem Bild angibt, der einem anatomischen Merkmal oder Untermerkmal des Subjekts entspricht,
Bestimmen (302), ob genügend Punkte empfangen worden sind, um eine Messung durchzuführen, basierend auf einem Vergleich von anatomischen Merkmalen und/oder Untermerkmalen, die dem angegebenen Punkt und allen anderen angegebenen Punkten mit vorgespeicherten anatomischen Daten entsprechen, wobei die vorgespeicherten anatomischen Daten für zumindest eine Messung die anatomischen Merkmale und/oder Untermerkmale angeben, die erforderlich sind, um die zumindest eine Messung durchzuführen; und
wenn genügend Punkte empfangen wurden, um eine Messung durchzuführen, Durchführen (304) der Messung.

15. Computerprogrammprodukt, das ein computerlesbares Medium umfasst, wobei das computerlesbare Medium einen darin verkörperten computerlesbaren Code aufweist, wobei der computerlesbare Code so konfiguriert ist, dass bei Ausführung durch einen geeigneten Computer oder Prozessor der Computer oder Prozessor dazu veranlasst wird, das Verfahren nach Anspruch 14 durchzuführen.

## Revendications

1. Appareil destiné à être utilisé dans le traitement de données d'image relatives à un sujet, l'appareil comprenant :
une mémoire (104) comprenant des données d'instructions représentant un ensemble d'instructions ; et
un processeur (102) configuré pour communiquer avec la mémoire et pour exécuter l'ensemble d'instructions (106), **caractérisé en ce que** l'ensemble d'instructions, lorsqu'il est exécuté par le processeur, amène le processeur à :
en réponse à la réception d'une entrée indiquant un point (612, 615, 616, 613) dans une image correspondant à une caractéristique ou sous-caractéristique anatomique du sujet,
déterminer (302) si suffisamment de points pour effectuer une mesure (727, 713) ont été reçus sur la base d'une comparaison des caractéristiques et/ou sous-caractéristiques anatomiques correspondant au point indiqué et à tout autre point indiqué avec des données anatomiques préenregistrées, les données anatomiques préenregistrées indiquant pour au moins une mesure les caractéristiques et/ou sous-caractéristiques anatomiques requises pour effectuer la au moins une mesure ; et
si suffisamment de points pour effectuer une mesure ont été reçus, effectuer (304) la mesure.

2. Appareil selon la revendication 1, dans lequel le processeur (102) est en outre amené à déterminer à quelle caractéristique ou sous-caractéristique anatomique correspond le point sur la base d'une entrée reçue d'un utilisateur indiquant la caractéristique ou sous-caractéristique anatomique à laquelle correspond le point, ou en classant le point dans une classe correspondant à la caractéristique ou sous-caractéristique anatomique.

3. Appareil selon la revendication 1 ou 2, dans lequel le processeur (102) est en outre amené à déterminer à quelle caractéristique ou sous-caractéristique anatomique correspond le point en classant le point dans une classe correspondant à une caractéristique anatomique en utilisant une première partie de l'image autour du point et en utilisant la position du point par rapport à l'image, et
si la classe dans laquelle le point est classé comprend des sous-classes correspondant à des sous-caractéristiques de la caractéristique anatomique, classer le point dans une sous-classe de la classe en utilisant une seconde partie de l'image autour du point.

4. Appareil selon la revendication 3, dans lequel la classification du point est en outre basée sur au moins l'un des éléments suivants : la classification d'un point précédent ; la position du point dans l'image par rapport à un point précédemment classé; l'emplacement du point dans un ordre d'entrée de points par rapport à une séquence prédite d'entrée de points.

5. Appareil selon l'une quelconque des revendications 3 ou 4, dans lequel la fiabilité de la classification d'un point précédemment classé est mise à jour sur la base d'au moins l'un des éléments suivants : la classification du point ; la position du point dans l'image par rapport au point précédemment classé.

6. Appareil selon l'une quelconque des revendications 3 à 5, dans lequel le processeur (102) est en outre configuré pour :
si le point est classé dans la même classe ou la même sous-classe lorsque la classe est séparable en sous-classes, qu'un point précédemment classé, demander une entrée indiquant quelle classification doit être utilisée pour au moins l'un du point et du point précédemment classé ; et
en réponse à la réception d'une indication d'une classification à utiliser pour au moins l'un du point ou du point précédemment classé, mettre à jour la classification d'au moins l'un du point ou du point précédemment classé sur la base de l'indication reçue.

7. Appareil selon l'une quelconque des revendications 3 à 6, dans lequel la classification est effectuée à l'aide d'un algorithme de classification qui a été formé à l'aide de parties d'images de formation autour de points correspondant à au moins l'une des caractéristiques anatomiques et des sous-caractéristiques anatomiques.

8. Appareil selon l'une quelconque des revendications 3 à 7, dans lequel la première partie de l'image est plus grande que la seconde partie de l'image.

9. Appareil selon une quelconque revendication précédente, dans lequel la mesure est effectuée en utilisant la position du point dans l'image.

10. Appareil selon une quelconque revendication précédente, dans lequel le processeur (102) est en outre configuré pour au moins l'une des opérations suivantes : stocker le point avec une étiquette correspondant à la caractéristique ou sous-caractéristique anatomique ; provoquer l'affichage du point sur un écran montrant l'image ; lorsque le point correspond à la symphyse pubienne, provoquer l'affichage d'une ligne horizontale passant par le point sur un écran montrant l'image.

11. Appareil selon une quelconque revendication précédente, dans lequel au moins l'une des suivantes : la caractéristique ou sous-caractéristique anatomique est une caractéristique ou sous-caractéristique utilisable pour évaluer un plancher pelvien du sujet ; la mesure est une mesure utilisable pour évaluer un plancher pelvien du sujet.

12. Appareil selon une quelconque revendication précédente, dans lequel les données d'image sont l'une quelconque d'une image ultrasonore et d'une image par résonance magnétique du sujet.

13. Système d'imagerie ultrasonore (200) comprenant l'appareil (100) selon l'une quelconque des revendications précédentes et un transducteur ultrasonore (4, 6) grâce auquel peuvent être obtenue des données d'image relatives à l'image.

14. Procédé mis en oeuvre par ordinateur (300) destiné à être utilisé dans le traitement de données d'image relatives à un sujet, le procédé comprenant :
en réponse à la réception d'une entrée indiquant un point dans une image correspondant à une caractéristique ou à une sous-caractéristique anatomique du sujet,
la détermination (302) si suffisamment de points pour effectuer une mesure ont été reçus sur la base d'une comparaison des caractéristiques et/ou sous-caractéristiques anatomiques correspondant au point indiqué et à tout autre point indiqué avec des données anatomiques préenregistrées, des données anatomiques préenregistrées indiquant pour au moins une mesure les caractéristiques et/ou sous-caractéristiques anatomiques requises pour effectuer la au moins une mesure ; et
si suffisamment de points pour effectuer une mesure ont été reçus, l'exécution (304) de la mesure.

15. Produit de programme informatique comprenant un support lisible par ordinateur, le support lisible par ordinateur présentant un code lisible par ordinateur incorporé dans celui-ci, le code lisible par ordinateur étant configuré de telle sorte que, lors de son exécution par un ordinateur ou un processeur approprié, l'ordinateur ou le processeur soit amené à exécuter le procédé selon la revendication 14.
